# EUROPÄISCHE PATENTSCHRIFT

(11) **EP 3 082 975 B1**
(45) Veröffentlichungstag und Bekanntmachung des Hinweises auf die Patenterteilung: **10.01.2018**
(21) Anmeldenummer: 14796129.6
(22) Anmeldetag: 12.11.2014
(51) Int. Cl.: A61Q 19/10, A61K 8/46

(54) **MILDES KOSMETISCHES REINIGUNGSMITTEL**
MILD COSMETIC CLEANSING COMPOSITION
COMPOSITION COSMETIQUE DOUCE NETTOYANTE

(30) Priorität: 17.12.2013 DE 102013226281
(43) Veröffentlichungstag der Anmeldung: 26.10.2016
(73) Patentinhaber: Henkel AG & Co. KGaA, 40589 Düsseldorf (DE)
(72) Erfinder: SCHRODER, Thomas, 22395 Hamburg (DE); HENTRICH, Dirk, 22457 Hamburg (DE)
(86) Internationale Anmeldenummer: PCT/EP2014/074302
(87) Internationale Veröffentlichungsnummer: WO 2015/090737

(56) Entgegenhaltungen:
- WO-A1-2013/089066
- WO-A1-2013/093473
- WO-A2-2011/117650
- SG-A1- 185 165

## Beschreibung

Die Erfindung liegt auf dem Gebiet der Kosmetik und betrifft milde Reinigungsmittel, die eine spezielle Tensidmischung enthalten.

Kosmetische Reinigungsmittel, wie beispielsweise Haarshampoos, basieren auf klassischen anionischen, amphoteren, zwitterionischen, nichtionischen und/oder kationischen Tensiden.

Aufgrund ihres hervorragenden Reinigungs- und Schaumvermögens werden überwiegend anionische Tenside, gegebenenfalls in der Mischung mit geringen Mengen an Co-Tensiden, eingesetzt.

Typische anionische Tenside, die in einer Vielzahl im Handel erhältlicher Shampoos eingesetzt werden, sind Alkylsulfate oder Alkylethersulfate.

Alkylethersulfate werden üblicherweise bevorzugt, da sie milder sind und über ein ausgezeichnetes Schaumvermögen verfügen.

Bei der Formulierung besonders milder Reinigungszusammensetzungen für die Anwendung auf sensiblen Hautpartien (wie beispielsweise der Gesichtshaut), für die Anwendung auf Babyhaut oder für die Anwendung auf sensiblen und/oder geschädigten Haaren sind Alkylethersulfate nicht immer zufrieden stellend, denn sie können ein für diese Anwendungen zu hohes Reizpotential aufweisen und geschädigten Haaren eine erhöhte Sprödigkeit verleihen.

In der Vergangenheit wurden daher zahlreiche Versuche unternommen, besonders milde Tensidmischungen zu finden, die ausreichend hohe Schaummengen und - qualitäten aufweisen, und die kein oder nur ein geringes Reizpotential auf der Haut und/oder der Schleimhaut hervorrufen. Die Tensidmischungen sollten sich zudem für die Verwendung in Haarbehandlungsmitteln, speziell für die Verwendung in Haarreinigungsmittel mit guten Pflegeeigenschaften eignen.

In der Anmeldung WO 92/084440 werden milde Tensidmischungen mit hervorragenden Schaumeigenschaften offenbart, die eine Mischung aus Acylisethionaten, zwitterionischen Tensiden und Alkylethersulfaten enthalten.

WO 11/015857 offenbart Reinigungszusammensetzungen mit niedrigem Irritationspotential auf der Haut, die neuartige C₅₋₃₀-Alkoyl-Alkyl-Isethionate und amphotere Tenside in einem Gewichtsverhältnis von 4 : 1 bis 1 : 4 enthalten. Die milden Reinigungsmittel eignen sich für eine Anwendung als Baby-Shampoo.

Nachteilig an einer Vielzahl milder Haut- und Haarreinigungsmittel ist, dass deren bessere Hautverträglichkeit oftmals zulasten der Textur der Reinigungsmittel geht.

Weiterhin nachteilig sind die oftmals unbefriedigenden Schaummengen und -eigenschaften, die mit milden Haarreinigungsmitteln erzielt werden können.

Darüber hinaus konnte beobachtet werden, dass die Pflegeeigenschaften milder Reinigungsmittel (insbesondere auf den Haaren) nicht immer zufrieden stellend sind.

Der vorliegenden Erfindung lag daher die Aufgabe zugrunde, besonders milde kosmetische Reinigungsmittel herzustellen, die gut haut- und schleimhautverträglich sind.

Die Reinigungsmittel sollten eine anwenderfreundliche Textur aufweisen und in Verbindung mit Wasser eine hohe Schaummenge erzeugen. Weiterhin sollten die Reinigungsmittel verbesserte Pflegeeigenschaften aufweisen.

Ein erster Gegenstand der Erfindung ist ein kosmetisches Reinigungsmittel, das in einem geeigneten Träger
a) mindestens ein anionisches Tensid gemäß der nachfolgenden Formel (I), in der die Reste R² bis R⁵ jeweils für ein Wasserstoffatom stehen, und
b) mindestens ein anionisches Tensid gemäß der nachfolgenden Formel (I) enthält, in der mindestens einer der Reste R² bis R⁵ für einen C₁-C₄-Alkylrest, und die anderen Reste unabhängig voneinander für ein Wasserstoffatom oder einen C₁-C₄-Alkylrest stehen, 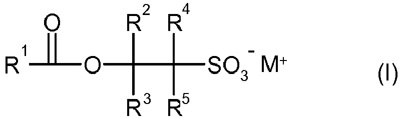 wobei
   - R¹ jeweils für einen linearen oder verzweigten, gesättigten oder ungesättigten Alkylrest mit 6 bis 30 Kohlenstoffatomen steht, und
   - M⁺ jeweils für ein Ammonium-, ein Alkanolammonium oder ein Metallkation steht.

Unter einem geeigneten Träger wird bevorzugt ein wässriger oder wässrig-alkoholischer Träger verstanden.

Bevorzugt enthält der Träger mindestens 50 Gew.-%, mehr bevorzugt mindestens 60 Gew.-% und besonders bevorzugt mindestens 70 Gew.-% Wasser.

Weiterhin kann der kosmetische Träger 0,01 bis 40 Gew.-%, bevorzugt 0,05 bis 35 Gew.-% und insbesondere 0,1 bis 30 Gew.-% mindestens eines Alkohols enthalten, der ausgewählt sein kann aus Ethanol, 1-Propanol, 2-Propanol, Isopropanol, Glycerin, Diglycerin, Triglycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycole, Sorbitol, Sorbitan, Benzylalkohol, Phenoxyethanol oder Mischungen dieser Alkohole.

Bevorzugt sind die wasserlöslichen Alkohole.

Besonders bevorzugt sind Ethanol, 1-Propanol, 2-Propanol, Isopropanol, 1,2-Propylenglycol, Glycerin, Benzylalkohol und/oder Phenoxyethanol sowie Mischungen dieser Alkohole. Insbesondere bevorzugt ist Glycerin.

Besonders milde kosmetische Reinigungsmittel mit gutem Schaumverhalten konnten hergestellt werden, wenn die Tenside a) und b) in einem bestimmten Gewichtsverhältnis a): b) von etwa 1 : 2,5 bis 4 : 1 eingesetzt wurden.

In einer ersten bevorzugten Ausführungsform sind erfindungsgemäße kosmetische Reinigungsmittel dadurch gekennzeichnet, dass sie die Tenside a) und b) bevorzugt in einem Gewichtsverhältnis a) zu b) von 1 : 2,5 bis 4 : 1, mehr bevorzugt von 1 : 2 bis 3 : 1, besonders bevorzugt von 1 : 1,5 bis 2,5 : 1 und insbesondere bevorzugt von 1 : 1 bis 2 : 1 enthalten.

Bevorzugte anionische Tenside a) der zuvor genannten Formel (I) weisen als Rest R¹ einen linearen oder verzweigten, gesättigten oder ungesättigten Alkylrest mit 8 bis 18 Kohlenstoffatomen auf. Besonders bevorzugt steht der Rest R¹ für einen C₈-, C₁₀-, C₁₂-, C₁₄-, C₁₆-Rest oder für Gemische dieser Fettsäurereste wie sie erhalten werden, wenn sich die Fettsäure(n) aus natürlichen Ölen wie beispielsweise Kokosöl ableitet(n).

M⁺ steht in bevorzugten anionischen Tensiden a) gemäß Formel (I) bevorzugt für ein Alkalimetallkation oder ein Ammoniumion, besonders bevorzugt für ein Kalium- oder ein Natriumion und insbesondere bevorzugt für ein Natriumion.

Ganz besonders bevorzugte anionische Tenside a) gemäß der zuvor genannten Formel (I) sind die unter den INCI-Bezeichnungen Natrium Cocoyl Isethionate, Kalium Cocoyl Isethionate, Ammonium Cocoyl Isethionate, Natrium Lauroyl Isethionate, Kalium Lauroyl Isethionate, Ammonium Lauroyl Isethionate, Natrium Myristoyl Isethionate, Kalium Methyl Isethionate und Ammonium Myristoyl Isethionate bekannten Verbindungen.

Insbesondere bevorzugt sind Natrium Cocoyl Isethionate und/oder Natrium Lauroyl Isethionate.

Entsprechende Handelsprodukte sind beispielsweise von den Firmen Clariant oder Uniquema unter den Handelsbezeichnungen "Hostapon®" oder "Arlatone®" erhältlich.

Bevorzugte anionische Tenside b) der zuvor genannten Formel (I) weisen als Rest R¹ einen linearen oder verzweigten, gesättigten oder ungesättigten Alkylrest mit 8 bis 18 Kohlenstoffatomen auf. Besonders bevorzugt steht der Rest R¹ für einen C₈-, C₁₀-, C₁₂-, C₁₄-, C₁₆-Rest oder für Gemische dieser Fettsäurereste wie sie erhalten werden, wenn sich die Fettsäure(n) aus natürlichen Ölen wie beispielsweise Kokosöl ableitet(n).

M⁺ steht in bevorzugten anionischen Tensiden a) gemäß Formel (I) bevorzugt für ein Alkalimetallkation oder ein Ammoniumion, besonders bevorzugt für ein Kalium- oder ein Natriumion und insbesondere bevorzugt für ein Natriumion.

In besonders bevorzugten anionischen Tensiden b) gemäß der zuvor genannten Formel (I) stehen die Reste R² bis R⁵ jeweils für eine Methyl-, Ethyl-, n-Propyl-, n-Butyl- oder 2-Butylgruppe.

Bevorzugt steht mindestens einer der Reste für R² bis R⁵ für eine Methyl-, Ethyl- oder eine n-Propylgruppe und insbesondere für eine Methylgruppe.

In einer besonders bevorzugten Ausführungsform steht nur einer der Reste R² bis R⁵ für eine C₁-C₄-Alkylgruppe - insbesondere eine Methylgruppe - und die anderen Reste stehen jeweils für ein Wasserstoffatom.

Prinzipiell ist es auch möglich, dass das anionische Tensid b) nach Formel (I) ein Isomerengemisch enthält, in dem sowohl Komponenten enthalten sind, die beispielsweise als Rest R² eine C₁-C₄-Alkylgruppe - insbesondere eine Methylgruppe - und als Reste R³ bis R⁵ jeweils ein Wasserstoffatom aufweisen, als auch Komponenten, die beispielsweise als Rest R⁵ eine C₁-C₄-Alkylgruppe - insbesondere eine Methylgruppe - und als Reste R² bis R⁴ jeweils ein Wasserstoffatom aufweisen.

Ganz besonders bevorzugte anionische Tenside b) der zuvor genannten Formel (I) sind die unter den INCI-Bezeichnungen Natrium Cocoyl Methyl Isethionate, Kalium Cocoyl Methyl Isethionate, Ammonium Cocoyl Methyl Isethionate, Natrium Lauroyl Methyl Isethionate, Kalium Lauroyl Methyl Isethionate, Ammonium Lauroyl Methyl Isethionate, Natrium Myristoyl Methyl Isethionate, Kalium Myristoyl Methyl Isethionate und Ammonium Myristoyl Methyl Isethionate bekannten Verbindungen.

Insbesondere bevorzugt sind Natrium Cocoyl Methyl Isethionate und/oder Natrium Lauroyl Methyl Isethionate. Entsprechende Handelsprodukte sind beispielsweise von der Firma Innospec unter der Handelsbezeichnung "Iselux® LQ-CLR-SB" erhältlich.

In einer zweiten bevorzugten Ausführungsform sind erfindungsgemäße kosmetische Reinigungsmittel dadurch gekennzeichnet, dass sie
a) mindestens ein anionisches Tensid gemäß Formel (I) enthalten, in der die Reste R² bis R⁵ jeweils für ein Wasserstoffatom stehen,
b) mindestens ein anionisches Tensid gemäß Formel (I) enthalten, in der mindestens einer der Reste R² bis R⁵ für einen Methylrest, und die anderen Reste für ein Wasserstoffatom stehen, mit der Maßgabe, dass
   - R¹ jeweils für einen linearen oder verzweigten, gesättigten oder ungesättigten Alkylrest mit 8 bis 18 Kohlenstoffatomen steht, und
   - M⁺ jeweils für ein Natrium-, Kalium- oder Ammoniumion steht.

Innerhalb dieser Ausführungsform sind erfindungsgemäße kosmetische Reinigungsmittel besonders bevorzugt, die
a) mindestens eines der unter den INCI-Bezeichnungen Sodium Cocoyl Isethionate oder Sodium Lauroyl Isethionate bekannten anionischen Tenside, und
b) mindestens eines der unter den INCI-Bezeichnungen Sodium Lauroyl Methyl Isethionate oder Sodium Cocoyl Methyl Isethionate bekannten anionischen Tenside enthalten.

Die erfindungsgemäße kosmetischen Reinigungsmittel können die Tenside a) und b) jeweils in Mengen von 0,5 bis 20 Gew.-% enthalten, wobei sich die Mengenangaben auf das Gesamtgewicht des Reinigungsmittels beziehen.

Werden die Tenside a) und b) jeweils in Mengen von weniger als 0,5 Gew.-% eingesetzt, so bilden die kosmetischen Reinigungsmittel keine ausreichend hohe Schaummenge. Werden die Tenside a) und b) jeweils in Mengen von mehr als 20 Gew.-% eingesetzt, so sind die Reinigungsmittel nicht mehr ausreichend hautschonend.

In einer dritten bevorzugten Ausführungsform sind erfindungsgemäße kosmetische Reinigungsmittel demnach dadurch gekennzeichnet, dass der Gewichtsanteil der anionischen Tenside a) und b) am Gesamtgewicht der Zusammensetzung bevorzugt jeweils 0,5 bis 20 Gew.-%, mehr bevorzugt 0,75 bis 15 Gew.-%, besonders bevorzugt 1 bis 10 Gew.-% und insbesondere 1,5 bis 7,5 Gew.-% beträgt.

Ein Ziel der erfindungsgemäßen Verwendung war es besonders milde, schaumstarke kosmetische Zusammensetzungen bereitzustellen, was durch die Kombination der Tenside a) und b) erreicht werden konnte.

Die durch die erfindungsgemäßen Mittel erzeugte Schaummenge entspricht in etwa der Schaummenge, die mit Alkylethersulfat-basierten Reinigungsmitteln erzielt werden kann, wobei die erfindungsgemäßen Mittel eine höhere Milde aufweisen.

Die zusätzliche Verwendung anionischer Alkylethersulfate in den erfindungsgemäßen Reinigungsmitteln ist daher nicht notwendig.

In einer weiteren bevorzugten Ausführungsform der Erfindung sind erfindungsgemäße kosmetische Reinigungsmittel daher dadurch gekennzeichnet, dass der Gewichtsanteil an Sulfatgruppen enthaltenden Tensiden am Gesamtgewicht der Zusammensetzungen bevorzugt weniger als 0,5 Gew.-%, mehr bevorzugt weniger als 0,3 Gew.-% und insbesondere weniger als 0,2 Gew.-% beträgt.

Die Schaummenge, die Schaumeigenschaften (insbesondere die hohe Schaumdichte) und/oder die Milde der erfindungsgemäßen Zusammensetzungen konnten hingegen noch weiter gesteigert werden, wenn ihnen spezielle amphotere Tenside hinzugefügt wurden.

In einer vierten bevorzugten Ausführungsform sind erfindungsgemäße kosmetische Reinigungsmittel demnach dadurch gekennzeichnet, dass sie zusätzlich mindestens ein amphoteres Tensid der nachfolgenden Formel (II) enthalten, 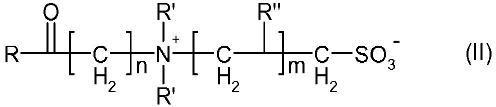 in der
- R für einen linearen oder verzweigten, gesättigten oder ungesättigten Alkylrest mit 8 bis 30 Kohlenstoffatomen steht,
- R' für einen C₁-C₆-Alkyl- oder für einen C₁-C₆-Hydroxyalkylrest steht,
- R" für ein Wasserstoffatom, eine Hydroxylgruppe, einen C₁-C₆-Alkyl- oder für einen C₁-C₆-Hydroxyalkylrest steht, und
- die Zahlen n und m unabhängig voneinander für Zahlen von 1 bis 5 stehen,
wobei der Gewichtsanteil des Tensids nach Formel (II) am Gesamtgewicht der Zusammensetzung bevorzugt 1 bis 15 Gew.-% beträgt.

Besonders bevorzugt beträgt der Gewichtsanteil des Tensids nach Formel (II) am Gesamtgewicht der Zusammensetzung 2 bis 12,5 Gew.-% und ganz besonders bevorzugt 3 bis 10 Gew.-%.

Innerhalb dieser vierten bevorzugten Ausführungsform sind kosmetische Reinigungsmittel besonders bevorzugt, die mindestens ein amphoteres Tensid der nachfolgenden Formel (II) enthalten, in der
- R für einen linearen oder verzweigten, gesättigten oder ungesättigten Alkylrest mit 8 bis 18 Kohlenstoffatomen steht,
- R' für einen Methyl- oder Ethylrest, bevorzugt für einen Methylrest, steht,
- R" für ein Wasserstoffatom oder für eine Hydroxylgruppe, bevorzugt für eine Hydroxylgruppe steht, und
- n für eine Zahl von 1 bis 3, bevorzugt für die Zahl 3, und m für die Zahlen 1 oder 2, bevorzugt für die Zahl 1 steht.

Insbesondere bevorzugte amphotere Tenside gemäß Formel (II) sind unter der INCI-Bezeichnung Cocamidopropyl Hydroxysultaine bekannt und im Handel erhältlich, beispielsweise unter der Bezeichnung "Mirataine®" von der Firma Rhodia, "Rewoteric®" von der Firma Goldschmidt oder "Mackam®" von der Firma The Mclntyre Group.

In einer fünften bevorzugten Ausführungsform sind erfindungsgemäße kosmetische Reinigungsmittel demnach dadurch gekennzeichnet, dass sie mindestens ein anionisches Tensid a) gemäß Formel (I), mindestens ein anionisches Tensid b) gemäß Formel (I) sowie mindestens ein amphoteres Tensid gemäß Formel (II) enthalten, wobei
- der Gewichtsanteil des Tensids a) am Gesamtgewicht der Reinigungsmittel bevorzugt 0,5 bis 20 Gew.-%, mehr bevorzugt 0,75 bis 15 Gew.-%,
- der Gewichtsanteil des Tensids b) am Gesamtgewicht der Reinigungsmittel bevorzugt 0,5 bis 20 Gew.-%, mehr bevorzugt 0,75 bis 15 Gew.-%, und
- der Gewichtsanteil des Tensids gemäß Formel (II) am Gesamtgewicht der Reinigungsmittel bevorzugt 1 bis 15 Gew.-%, mehr bevorzugt 2 bis 12,5 Gew.-%
beträgt.

Insbesondere bevorzugte kosmetische Reinigungsmittel innerhalb dieser Ausführungsform sind dadurch gekennzeichnet, dass sie
a) mindestens eines der unter den INCI-Bezeichnungen Sodium Cocoyl Isethionate oder Sodium Lauroyl Isethionate bekannten anionischen Tenside,
b) mindestens eines der unter den INCI-Bezeichnungen Sodium Lauroyl Methyl Isethionate oder Sodium Cocoyl Methyl Isethionate bekannten anionischen Tenside, und
c) mindestens ein unter der INCI-Bezeichnung Cocamidopropyl Hydroxysultaine bekanntes amphoteres Tensid enthalten,
wobei
- der Gewichtsanteil des Sodium Cocoyl Isethionats oder des Sodium Lauroyl Isethionats am Gesamtgewicht der Reinigungsmittel bevorzugt 1 bis 10 Gew.-% und insbesondere 1,5 bis 7,5 Gew.-% beträgt,
- der Gewichtsanteil des Sodium Lauroyl Methyl Isethionats oder des Sodium Cocoyl Methyl Isethionats am Gesamtgewicht der Reinigungsmittel bevorzugt 1 bis 10 Gew.-% und insbesondere 1,5 bis 7,5 Gew.-% beträgt, und
- der Gewichtsanteil des Cocamidopropyl Hydroxysultains am Gesamtgewicht der Reinigungsmittel bevorzugt 3 bis 10 Gew.-% beträgt.

Neben der Schaummenge, den Schaumeigenschaften (insbesondere die hohe Schaumdichte) und/oder der Milde konnten die Pflegeeigenschaften der erfindungsgemäßen Zusammensetzungen weiter gesteigert werden, wenn ihnen mindestens ein Alkyl(oligo)glucosid hinzugefügt wurde.

In einer sechsten bevorzugten Ausführungsform sind erfindungsgemäße kosmetische Reinigungsmittel demnach dadurch gekennzeichnet, dass sie zusätzlich mindestens ein Alkyl(oligo)glycosid der allgemeinen Formel RO-[G]ₓ enthalten, in der R für einen Alkyl- und/oder Alkenylrest mit 4 bis 24 C-Atomen, G für einen Zuckerrest mit 5 oder 6 C-Atomen und x für Zahlen von 1 bis 10 steht, wobei der Gewichtsanteil des Alkyl(oligo)glycosids am Gesamtgewicht der Reinigungsmittel bevorzugt 0,5 bis 10 Gew.-% beträgt.

Bevorzugte Alkyl(oligo)glycoside können ausgewählt sein aus Verbindungen der allgemeinen Formel der RO-[G]ₓ, in denen sich [G] bevorzugt von Aldosen und/oder Ketosen mit 5-6 Kohlenstoffatomen, vorzugsweise von Glucose ableitet, und in denen der Rest R bevorzugt für einen Alkylrest mit 8, 10, 12, 14, 16 und/oder 18 Kohlenstoffatomen steht.

Die Indexzahl x steht für den Oligomerisierungsgrad (DP), d.h. für die Verteilung der Mono- und Oligoglycoside. Die Indexzahl x weist vorzugsweise einen Wert im Bereich von 1 bis 10, besonders bevorzugt im Bereich von 1 bis 3 auf, wobei es sich dabei um keine ganze Zahl, sondern um eine gebrochene Zahl handeln kann, die analytisch ermittelt werden kann.

Besonders bevorzugte Alkyl(oligo)glycoside weisen einen Oligomerisierungsgrad zwischen 1,2 und 1,5 auf.

Insbesondere bevorzugte Alkyl(oligo)glycoside sind die unter den INCI-Bezeichnungen Caprylyl/Capryl Glucoside, Decyl Glucoside, Lauryl Glucoside und Coco Glucoside bekannten Verbindungen.

Besonders bevorzugt beträgt der Gewichtsanteil der zuvor genannten Alkyl(oligo)glycoside am Gesamtgewicht der Zusammensetzung 0,5 bis 7,5 Gew.-% und ganz besonders bevorzugt 0,5 bis 5 Gew.-%.

In einer siebten bevorzugten Ausführungsform sind erfindungsgemäße kosmetische Reinigungsmittel dadurch gekennzeichnet, dass sie mindestens ein anionisches Tensid a) gemäß Formel (I), mindestens ein anionisches Tensid b) gemäß Formel (I), mindestens ein amphoteres Tensid gemäß Formel (II) sowie mindestens ein Alkyl(oligo)glycosid enthalten, wobei
- der Gewichtsanteil des Tensids a) am Gesamtgewicht der Reinigungsmittel bevorzugt 0,5 bis 20 Gew.-%, mehr bevorzugt 0,75 bis 15 Gew.-%,
- der Gewichtsanteil des Tensids b) am Gesamtgewicht der Reinigungsmittel bevorzugt 0,5 bis 20 Gew.-%, mehr bevorzugt 0,75 bis 15 Gew.-%,
- der Gewichtsanteil des Tensids gemäß Formel (II) am Gesamtgewicht der Reinigungsmittel bevorzugt 1 bis 15 Gew.-%, mehr bevorzugt 2 bis 12,5 Gew.-%, und
- der Gewichtsanteil des Alkyl(oligo)glycosids am Gesamtgewicht der Reinigungsmittel bevorzugt 0,5 bis 10 Gew.-%, mehr bevorzugt 0,5 bis 7,5 Gew.-% beträgt.

Insbesondere bevorzugte kosmetische Reinigungsmittel innerhalb dieser Ausführungsform sind dadurch gekennzeichnet, dass sie
a) mindestens eines der unter den INCI-Bezeichnungen Sodium Cocoyl Isethionate oder Sodium Lauroyl Isethionate bekannten anionischen Tenside,
b) mindestens eines der unter den INCI-Bezeichnungen Sodium Lauroyl Methyl Isethionate oder Sodium Cocoyl Methyl Isethionate bekannten anionischen Tenside,
c) mindestens ein unter der INCI-Bezeichnung Cocamidopropyl Hydroxysultaine bekanntes amphoteres Tensid und
d) mindestens eines der unter den INCI-Bezeichnungen Caprylyl/Capryl Glucoside, Decyl Glucoside, Lauryl Glucoside und Coco Glucoside bekannten Verbindungen enthalten,
wobei
- der Gewichtsanteil des Sodium Cocoyl Isethionats oder des Sodium Lauroyl Isethionats am Gesamtgewicht der Reinigungsmittel bevorzugt 1 bis 10 Gew.-% und insbesondere 1,5 bis 7,5 Gew.-% beträgt,
- der Gewichtsanteil des Sodium Lauroyl Methyl Isethionats oder des Sodium Cocoyl Methyl Isethionats am Gesamtgewicht der Reinigungsmittel bevorzugt 1 bis 10 Gew.-% und insbesondere 1,5 bis 7,5 Gew.-% beträgt,
- der Gewichtsanteil des Cocamidopropyl Hydroxysultains am Gesamtgewicht der Reinigungsmittel bevorzugt 3 bis 10 Gew.-%, und
- der Gewichtsanteil des Caprylyl/Capryl Glucosids, Decyl Glucosids, Lauryl Glucosids oder des Coco Glucosids am Gesamtgewicht der Reinigungsmittel bevorzugt 0,5 bis 5 Gew.-% beträgt.

In einer achten bevorzugten Ausführungsform sind erfindungsgemäße kosmetische Reinigungsmittel dadurch gekennzeichnet, dass sie zusätzlich mindestens ein Chelatisierungsmittel enthalten, wobei der Gewichtsanteil des Chelatisierungsmittels am Gesamtgewicht der Zusammensetzung bevorzugt 0,01 bis 3 Gew.-%, mehr bevorzugt 0,05 bis 2 Gew.-% und insbesondere 0,1 bis 1 Gew.-% beträgt.

Geeignete Chelatisierungsmittel können bevorzugt ausgewählt sein aus Aminocarboxylaten, Aminophosphonaten, Milchsäure, Weinsäure, Zitronensäure oder Mischungen davon.

Besonders bevorzugt sind Ethylendiamintetraessigsäure (EDTA) und/oder Ethylendiamindisuccinat (EDDS). Insbesondere bevorzugt aufgrund seiner biologischen Abbaubarkeit ist EDDS.

In einer weiteren bevorzugten Ausführungsform können die pflegenden Eigenschaften der kosmetischen Mittel weiter gesteigert werden, wenn sie zusätzlich mindestens einen konditionierenden Wirkstoff enthalten, der ausgewählt sein kann aus der Gruppe der
- Proteinhydrolysate,
- kationischen Polymere,
- Vitamine,
- Fette, Öle und/oder Wachse,
- Glycerin.

Unter geeigneten Proteinhydrolysaten sind Produktgemische zu verstehen, die durch sauer, basisch oder enzymatisch katalysierten Abbau von Proteinen (Eiweißen) erhalten werden können.

Es können Proteinhydrolysate pflanzlichen, tierischen und/oder marinen Ursprungs eingesetzt werden. Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Der Gewichtsanteil des oder der Proteinhydrolysats(e) am Gesamtgewicht der kosmetischen Mittel beträgt bevorzugt 0,01 bis 5 Gew.-%, mehr bevorzugt 0,025 bis 3 Gew.-% und insbesondere 0,05 bis 2 Gew.-%.

Geeignete kationische Polymere sind beispielsweise:
- quaternisierte Cellulose-Derivate, wie sie unter den Bezeichnungen Celquat® und Polymer JR® im Handel erhältlich sind,
- quaternisierte Cellulose-Derivate, die hydrophob modifiziert sein können, beispielsweise Polyquaternium-67,
- kationische Alkylpolyglycoside,
- kationisierter Honig, beispielsweise das Handelsprodukt Honeyquat® 50,
- kationische Guar-Derivate, wie insbesondere die unter den Handelsnamen Cosmedia®Guar N-Hance® und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Bevorzugte weitere kationische Polymere sind vorzugsweise ausgewählt aus den unter den INCI-Bezeichnungen Guar Hydroxypropyltrimonium Chloride, Polyquaternium-67, Polyquaternium-10, Polyquaterniu-6, Polyquaternium-7 und/oder Polyquaternium-37 bekannten Polymere. Insbesondere bevorzugt sind die unter den INCI-Bezeichnungen bekannten Polymere Guar Hydroxypropyltrimonium Chloride und Polyquaternium-10 bekannten Polymere.

Der Gewichtsanteil des oder der weiteren kationischen Polymeren am Gesamtgewicht der kosmetischen Mittel beträgt bevorzugt 0,01 bis 5 Gew.-%, mehr bevorzugt 0,025 bis 4 Gew.-%, besonders bevorzugt 0,05 bis 3 Gew.-% und insbesondere 0,1 bis 2 Gew.-%.

Unter geeigneten Vitaminen sind bevorzugt die folgenden Vitamine, Provitamine und Vitaminvorstufen sowie deren Derivate zu verstehen:
- Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
- Vitamin B: zur Vitamin B-Gruppe oder zu dem Vitamin B-Komplex gehören u. a.
   ➢ Vitamin B₁ (Thiamin)
   ➢ Vitamin B₂ (Riboflavin)
   ➢ Vitamin B₃. Unter dieser Bezeichnung werden häufig die Verbindungen Nicotinsäure und Nicotinsäureamid (Niacinamid) geführt.
   ➢ Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols, Pantolacton sowie kationisch derivatisierte Panthenole. Einzelne Vertreter sind beispielsweise das Panthenoltriacetat, der Panthenolmonoethylether und dessen Monoacetat sowie kationische Panthenolderivate.
   ➢ Vitamin B₆ (Pyridoxin sowie Pyridoxamin und Pyridoxal).
- Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol).
- Vitamin F: unter dem Begriff "Vitamin F" werden üblicherweise essentielle Fettsäuren, insbesondere Linolsäure, Linolensäure und Arachidonsäure, verstanden.
- Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Besonders bevorzugt sind Vitamine, Provitamine und Vitaminvorstufen aus den Gruppen A, B, E und H. Insbesondere bevorzugt sind Nicotinsäureamid, Biotin, Pantolacton und/oder Panthenol.

Der Gewichtsanteil des oder der Vitamins(e), Vitaminderivats(e), und/oder der Vitaminvorstufe(n) am Gesamtgewicht der kosmetischen Mittel beträgt bevorzugt 0,001 bis 2 Gew.-%, besonders bevorzugt 0,005 bis 1 Gew.-% und insbesondere 0,01 bis 0,5 Gew.-%.

Geeignete Öl-, Wachs- und/oder Fettkomponenten können bevorzugt ausgewählt sein aus mineralischen, natürlichen und synthetischen Ölkomponenten und/oder Fettstoffen.

Als natürliche (pflanzliche) Öle werden üblicherweise Triglyceride und Mischungen von Triglyceriden eingesetzt. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol® S).

Als Ölkomponente kann weiterhin ein Dialkylether dienen.

Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-isopentylether, Di-3-ethyldecylether, tert.-Butyl-n-octylether, iso-Pentyl-n-octylether und 2-Methylpentyl-n-octylether.

Besonders bevorzugt ist der Di-n-octylether, der im Handel unter der Bezeichnung Cetiol® OE erhältlich ist.

Als synthetische Öle kommen bevorzugt Silikonverbindungen in Betracht.

Silikone bewirken auf dem Haar ausgezeichnete konditionierende Eigenschaften. Insbesondere bewirken sie eine bessere Kämmbarkeit der Haare in nassem und trockenem Zustand und wirken sich in vielen Fällen positiv auf den Haargriff und die Weichheit der Haare aus.

Es ist daher erstrebenswert, in kosmetischen Haarbehandlungsmitteln Silikone einzusetzen. Geeignete Silikone können ausgewählt sein unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Unter Fettstoffen sind zu verstehen Fettsäuren, Fettalkohole sowie natürliche und synthetische Wachse, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂-Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
   Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Diisotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol® CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls® 90-018, Monomuls® 90-L12, Cetiol® HE oder Cutina® MD.

Der Gewichtsanteil der ÖI-, Wachs- und/oder Fettkomponenten am Gesamtgewicht der kosmetischen Mittel beträgt bevorzugt 0,01 bis 5 Gew.-%, besonders bevorzugt 0,025 bis 4 Gew.-% und insbesondere 0,05 bis 3 Gew.-%.

Besonders bevorzugt sind kosmetische Reinigungsmitteln, die als zusätzliche pflegende Komponente mindestens ein kationisches Polymer, mindestens eine wasserunlösliche Silikonverbindung und/oder mindestens ein Vitamin(derivat) enthalten.

Insbesondere bevorzugt ist ein pflegender Wirkstoffkomplex, der mindestens zwei Pflegestoffe aus den zuvor genannten Wirkstoffgruppen enthält.

Glycerin kann kosmetischen Mitteln separat in einer Menge von bis zu 10 Gew.-% (bezogen auf das Gesamtgewicht des Reinigungsmittels) zugegeben werden. Es kann aber auch Bestandteil des wässrigalkoholischen Trägers sein.

Es wurde festgestellt, dass die erfindungsgemäßen kosmetischen Mittel auch für eine Anwendung als Antischuppenzubereitung geeignet sind.

Das Gesamtgewicht an Antischuppenmitteln am Gesamtgewicht der kosmetischen Mittel kann bevorzugt 0,01 bis 10 Gew.-%, mehr bevorzugt 0,025 bis 7,5 Gew.-%, besonders bevorzugt 0,05 bis 5 Gew.-% und insbesondere 0,075 bis 3 Gew.-% betragen.

Geeignete Antischuppenwirkstoffe können ausgewählt sein aus Piroctone Olamine, Climbazol, Zink Pyrithion, Ketoconazole, Salicylsäure, Schwefel, Selensulfid, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten.

Bevorzugt sind Climbazol, Zink Pyrithion und Piroctone Olamine.

Weitere Wirk-, Hilfs- und Zusatzstoffe, die in den erfindungsgemäßen kosmetischen Mitteln bevorzugt enthalten sein können, sind beispielsweise:
- Pflanzenextrakte,
- Feuchthaltemittel,
- Parfums,
- UV-Filter,
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Verdickungsmittel wie Acryl- und Methacryl(co)polymere, beispielsweise die vernetzten Homopolymere der Acrylsäure (INCI- Bezeichnung: Carbomer), die auch als Carboxyvinylpolymere bezeichnet werden. Solche Polyacrylsäuren sind unter anderem von der Fa. 3V Sigma unter dem Handelsnamen Polygel®, z. B. Polygel DA und von der Fa. B. F. Goodrich unter dem Handelsnamen Carbopol® erhältlich, z. B. Carbopol 940 (Molekulargewicht ca. 4.000.000), Carbopol 941 (Molekulargewicht ca. 1. 250.000) oder Carbopol 934 (Molekulargewicht ca. 3. 000.000). Weiterhin sind beispielsweise folgende AcrylsäureCopolymere geeignet:
   a. Copolymere von zwei oder mehr Monomeren aus der Gruppe der Acrylsäure, Methacrylsäure und ihrer einfachen, vorzugsweise mit C1-C4-Alkanolen gebildeten, Ester (INCI-Bezeichnung: Acrylates Copolymer), zu denen etwa die Copolymere von Methacrylsäure, Butylacrylat und Methylmethacrylat oder von Butylacrylat und Methylmethacrylat gehören, und die beispielsweise von der Fa. Rohm & Haas unter den Handelsnamen Aculyn® und Acusol® sowie von der Firma Degussa (Goldschmidt) unter dem Handelsnamen Tego® Polymer erhältlich sind, zum Beispiel die anionischen nicht-assoziativen Polymere Aculyn 22, Aculyn 28, Aculyn 33 (vernetzt), Acusol 810, Acusol 820, Acusol 823 und Acusol 830;
   b. vernetzte hochmolekulare Acrylsäure-Copolymere, zu denen etwa die mit einem Allylether der Saccharose oder des Pentaerythrits vernetzten Copolymere von C10-C30-Alkylacrylaten mit einem oder mehreren Monomeren aus der Gruppe der Acrylsäure, Methacrylsäure und ihrer einfachen, vorzugsweise mit C1-C4-Alkanolen gebildeten, Ester (INCI-Bezeichnung: Acrylates-(C10-C30)-Alkyl Acrylate Crosspolymer) gehören, und die beispielsweise von der Fa. B. F. Goodrich unter dem Handelsnamen Carbopol® erhältlich sind, z. B. das hydrophobierte Carbopol ETD 2020 und Carbopol 1382 (INCI Acrylates-(C10-C30)-Alkyl Acrylate Crosspolymer) sowie Carbopol Aqua 30,
- Strukturanten wie Maleinsäure und Milchsäure,
- Dimethylisosorbid,
- Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Substanzen zur Einstellung des pH-Wertes, beispielsweise α- und β-Hydroxycarbonsäuren wie Citronensäure, Milchsäure, Äpfelsäure, Glycolsäure,
- Wirkstoffe wie Bisabolol,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,-
- Konservierungsmittel, wie beispielsweise Natriumbenzoat oder Salicylsäure,
- Zusätzliche Viskositätsregler wie Salze (NaCl).

Die erfindungsgemäßen kosmetischen Mittel können bevorzugt einen pH-Wert im Bereich von 3 bis 8, mehr bevorzugt von 3,5 bis 7,5, besonders bevorzugt von 4 bis 7 und insbesondere von 5 bis 6,5 aufweisen.

Die erfindungsgemäßen kosmetischen Mittel weisen bevorzugt eine Viskosität im Bereich von 1,000 bis 15,000 mPas, bevorzugt von 1,500 bis 12,500 mPas und insbesondere von 3,000 bis 10,000 mPas auf (jeweils gemessen mit einem Haake Rotationsviskosimeter VT550; 20 deg.C; Messeinrichtung MV; Spindel MV II; 8 UPM). Zusammensetzungen einer solchen Viskosität lassen sich gut auf der jeweiligen Anwendungsoberfläche - besonders bevorzugt auf den Haaren - verteilen und gegebenenfalls nach der Anwendung mit Wasser wieder abspülen.

### Beispiele:

Es wurden die folgenden erfindungsgemäßen Haarreinigungsmittel hergestellt (Zusammensetzungen A-D der nachfolgenden Tabelle; Mengenangaben in [Gew.-%]):

| | **A** | **B** | **C** | **D** |
|---|---|---|---|---|
| **Carbopol ETD 2020®¹** | 0,6 | | | |
| **Texapon N70®²** | | | | 10,0 |
| **Iselux LQ®³** | 10,0 | 6,0 | 10,0 | |
| **Hostapon SCI 85C®⁴** | | 4,0 | 4,0 | |
| **Rewoteric AM C®⁵** | 4,0 | 4,0 | 4,0 | |
| **Plantacare 818UP®⁶** | 1,5 | 1,5 | 1,5 | 1,5 |
| **Tego Betain F50®⁷** | | | | 5,5 |
| **Mirataine CBS®⁸** | 12,0 | 12,0 | 12,0 | |
| **Polymer JR 400®⁹** | 0,4 | 0,4 | 0,4 | 0,4 |
| **Styrene/Acrylates Copolymer** | 1,0 | 1,0 | 1,0 | 1,0 |
| **EDDS (Natrquest E30)** | 0,7 | 0,7 | 0,7 | |
| **Abil ME 45®¹⁰** | 1,0 | 1,0 | 1,0 | 1,0 |
| **Antil 200®¹¹** | 2,5 | 2,5 | 2,5 | 2,5 |
| **Konservierungsmittel** | q.s. | q.s. | q.s. | q.s. |
| **Parfum** | q.s. | q.s. | q.s. | q.s. |
| **Zitronensäure/NaOH (pH 5,5-6,5)** | q.s. | q.s. | q.s. | q.s. |
| **Wasser** | ad 100 | ad 100 | ad 100 | ad 100 |
| **Aussehen** | weiß, milchig | | | |
| **Viskosität*** | 4,000 - 8,000 mPas | | | |
| **Bestimmung der Schaummenge** | 3 | 2-3 | 2-3 | 2-3 |
| **Griff des Schaumes in nassem Haar** | 3 | 2 | 2-3 | 3 |
| **Reduktion der Nasskämmbarkeit [%]** | 35 | 43 | 51 | 38 |

| | | | | |
|---|---|---|---|---|
| * bestimmt mit einem Haake Rotationsviskosi-meter VT550; 20°C; Messeinrichtung MV; Spindel MV II; 8 UPM In den Zusammensetzungen A-D wurden die folgenden Handelsprodukte eingesetzt: ¹ INCI-Bezeichnung: ACRYLATES/C10-30 ALKYL ACRYLATE CROSSPOLYMER; Lubrizol ² INCI-Bezeichnung: SODIUM LAURETH SULFATE; 70% AS; BASF ³ INCI-Bezeichnung: SODIUM LAUROYL METHYL ISETHIONATE; 32%AS; Innospec, ⁴ INCI-Bezeichnung: DISODIUM COCOYL ISETHIONATE; 84% AS; Clariant ⁵ INCI-Bezeichnung: SODIUM COCOAMPHOACETATE; 50%AS; Evonik ⁶ INCI-Bezeichnung: COCO-GLUCOSIDE, AQUA; 51-53%AS; BASF, ⁷ INCI-Bezeichnung: AQUA, COCAMIDOPROPYL BETAINE; 35-37,5%AS; Evonik, ⁸ INCI-Bezeichnung: COCAMIDOPROPYL HYDROXYSULTAINE; 50% AS; Rhodia ⁹ INCI-Bezeichnung: Polyquaternium-10; Shanghai Jida meticulous Chemical Industry ¹⁰ INCI-Bezeichnung: SILICONE QUATERNIUM-22, POLYGLYCERYL-3 CAPRATE, DIPROPYLENE GLYCOL, COCAMIDOPROPYL BETAINE; Evonik, ¹¹ INCI-Bezeichnung: PEG-200 HYDROGENATED GLYCERYL PALMATE, PEG-7 GLYCERYL COCOATE; Evonik | | | | |

Die Ergebnisse in der Tabelle zeigen, dass die erfindungsgemäßen Zusammensetzungen (B, C) eine Schaummenge bilden, die vergleichbar ist mit den Schaummengen, die in klassichen Tensidsystemen für kosmetische Reinigungsmittel üblich ist (D). Die Schaumeigenschaften der erfindungsgemäßen Zusammensetzungen sind zudem besser als die Schaumeigenschaften vergleichbarer Zusammensetzungen, die nicht die Kombination der Tenside a) unnd b) enthalten.

Schließlich konnte die Nasskämmbarkeit von Haaren, die mit den erfindungsgemäßen Reinigungsmitteln behandelt wurden, signifikant verbessert werden.

## Patentansprüche

1. Kosmetisches Reinigungsmittel, enthaltend in einem geeigneten Träger
a) mindestens ein anionisches Tensid gemäß der nachfolgenden Formel (I), in der die Reste R² bis R⁵ jeweils für ein Wasserstoffatom stehen,
b) mindestens ein anionisches Tensid gemäß der nachfolgenden Formel (I), in der mindestens einer der Reste R² bis R⁵ für einen C₁-C₄-Alkylrest, und die anderen Reste unabhängig voneinander für ein Wasserstoffatom oder einen C₁-C₄-Alkylrest stehen, 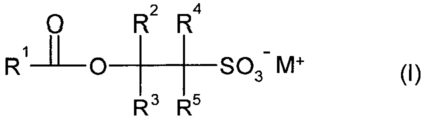 wobei
- R¹ jeweils für einen linearen oder verzweigten, gesättigten oder ungesättigten Alkylrest mit 6 bis 30 Kohlenstoffatomen steht, und
- M⁺ jeweils für ein Ammonium-, ein Alkanolammonium oder ein Metallkation steht, und
c) mindestens ein weiteres Tensid, ausgewählt aus
i. amphoteren Tensiden der nachfolgenden Formel (II), 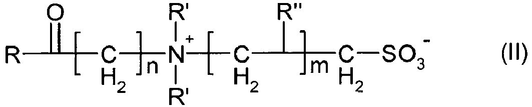 in der
- R für einen linearen oder verzweigten, gesättigten oder ungesättigten Alkylrest mit 8 bis 30 Kohlenstoffatomen steht,
- R' für einen C₁-C₆-Alkyl- oder für einen C₁-C₆-Hydroxyalkylrest steht,
- R" für ein Wasserstoffatom, eine Hydroxylgruppe, einen C₁-C₆-Alkyl- oder für einen C₁-C₆-Hydroxyalkylrest steht,
- die Zahlen n und m unabhängig voneinander für Zahlen von 1 bis 5 stehen, und/oder
ii. Alkyl(oligo)glycosiden der allgemeinen Formel RO-[G]ₓ, in der R für einen Alkyl-und/oder Alkenylrest mit 4 bis 24 C-Atomen, G für einen Zuckerrest mit 5 oder 6 C-Atomen und x für Zahlen von 1 bis 10 steht.

2. Kosmetisches Reinigungsmittel nach Anspruch 1, **dadurch gekennzeichnet, dass** es die Tenside a) und b) in einem Gewichtsverhältnis von 1 : 2,5 bis 4 : 1, bevorzugt von 1 : 2 bis 3 : 1, besonders bevorzugt von 1 : 1,5 bis 2,5 : 1 und insbesondere bevorzugt von 1 : 1 bis 2 : 1 enthält.

3. Kosmetisches Reinigungsmittel nach einem der vorherigen Ansprüche, **dadurch gekennzeichnet, dass** es
a) mindestens ein anionisches Tensid gemäß Formel (I) enthält, in der die Reste R² bis R⁵ jeweils für ein Wasserstoffatom stehen,
b) mindestens ein anionisches Tensid gemäß Formel (I) enthält, in der mindestens einer der Reste R² bis R⁵ für einen Methylrest, und die anderen Reste für ein Wasserstoffatom stehen,
mit der Maßgabe, dass
- R¹ jeweils für einen linearen oder verzweigten, gesättigten oder ungesättigten Alkylrest mit 8 bis 18 Kohlenstoffatomen steht, und
- M⁺ jeweils für ein Natrium-, Kalium- oder Ammoniumion steht.

4. Kosmetisches Reinigungsmittel nach einem der vorherigen Ansprüche, **dadurch gekennzeichnet, dass** es mindestens eines der unter den INCI-Bezeichnungen Sodium Cocoyl Isethionate oder Sodium Lauroyl Isethionate bekannten anionischen Tenside und mindestens eines der unter den INCI-Bezeichnungen Sodium Lauroyl Methyl Isethionate oder Sodium Cocoyl Methyl Isethionate bekannten anionischen Tenside enthält.

5. Kosmetisches Reinigungsmittel nach einem der vorherigen Ansprüche, **dadurch gekennzeichnet, dass** der Gewichtsanteil der anionischen Tenside a) und b) am Gesamtgewicht der Zusammensetzung 0,5 bis 20 Gew.-%, bevorzugt 0,75 bis 15 Gew.-%, besonders bevorzugt 1 bis 10 Gew.-% und insbesondere 1,5 bis 7,5 Gew.-% beträgt.

6. Kosmetisches Reinigungsmittel nach einem der vorherigen Ansprüche, **dadurch gekennzeichnet, dass** es ein amphoteres Tensid der Formel (II) in einem Gewichtsanteil von 1 bis 15 Gew.-% des am Gesamtgewicht der Zusammensetzung enthält.

7. Kosmetisches Reinigungsmittel nach Anspruch 1, **dadurch gekennzeichnet, dass** in dem Tensid gemäß Formel (11)
- R für einen linearen oder verzweigten, gesättigten oder ungesättigten Alkylrest mit 8 bis 18 Kohlenstoffatomen steht,
- R' für einen Methyl- oder Ethylrest, bevorzugt für einen Methylrest, steht,
- R" für ein Wasserstoffatom oder für eine Hydroxylgruppe, bevorzugt für eine Hydroxylgruppe steht, und
- n für eine Zahl von 1 bis 3, bevorzugt für die Zahl 3, und m für die Zahlen 1 oder 2, bevorzugt für die Zahl 1 steht.

8. Kosmetisches Reinigungsmittel nach einem der vorhergehenden Ansprüche, **dadurch gekennzeichnet, dass** sie mindestens ein anionisches Tensid a) gemäß Formel (I),
mindestens ein anionisches Tensid b) gemäß Formel (I) sowie mindestens ein amphoteres Tensid gemäß Formel (II) enthalten, wobei
- der Gewichtsanteil des Tensids a) am Gesamtgewicht der Reinigungsmittel bevorzugt 0,5 bis 20 Gew.-%, mehr bevorzugt 0,75 bis 15 Gew.-%,
- der Gewichtsanteil des Tensids b) am Gesamtgewicht der Reinigungsmittel bevorzugt 0,5 bis 20 Gew.-%, mehr bevorzugt 0,75 bis 15 Gew.-%, und
- der Gewichtsanteil des Tensids gemäß Formel (II) am Gesamtgewicht der Reinigungsmittel bevorzugt 1 bis 15 Gew.-%, mehr bevorzugt 2 bis 12,5 Gew.-% beträgt.

9. Kosmetisches Reinigungsmittel nach Anspruch 1, **dadurch gekennzeichnet, dass** es ein Alkyl(oligo)glycosid der allgemeinen Formel RO-[G]ₓ in einem Gewichtsanteil von 0,5 bis 10 Gew.-% am Gesamtgewicht der Zusammensetzung enthält.

10. Kosmetisches Reinigungsmittel nach einem der vorherigen Ansprüche, **dadurch gekennzeichnet, dass** es mindestens ein anionisches Tensid a) gemäß Formel (I),
mindestens ein anionisches Tensid b) gemäß Formel (I), mindestens ein amphoteres Tensid gemäß Formel (II) sowie mindestens ein Alkyl(oligo)glycosid enthält, wobei
- der Gewichtsanteil des Tensids a) am Gesamtgewicht der Reinigungsmittel bevorzugt 0,5 bis 20 Gew.-%, mehr bevorzugt 0,75 bis 15 Gew.-%,
- der Gewichtsanteil des Tensids b) am Gesamtgewicht der Reinigungsmittel bevorzugt 0,5 bis 20 Gew.-%, mehr bevorzugt 0,75 bis 15 Gew.-%,
- der Gewichtsanteil des Tensids gemäß Formel (II) am Gesamtgewicht der Reinigungsmittel bevorzugt 1 bis 15 Gew.-%, mehr bevorzugt 2 bis 12,5 Gew.-%, und
- der Gewichtsanteil des Alkyl(oligo)glycosids am Gesamtgewicht der Reinigungsmittel bevorzugt 0,5 bis 10 Gew.-%, mehr bevorzugt 0,5 bis 7,5 Gew.-% beträgt.

11. Kosmetisches Reinigungsmittel nach einem der vorherigen Ansprüche, **dadurch gekennzeichnet, dass** es zusätzlich mindestens ein Chelatisierungsmittel enthält, wobei der Gewichtsanteil des Chelatisierungsmittels am Gesamtgewicht der Zusammensetzung 0,01 bis 3 Gew.-%, bevorzugt 0,05 bis 2 Gew.-% und insbesondere 0,1 bis 1 Gew.-% beträgt.

12. Kosmetisches Reinigungsmittel nach Anspruch 11, **dadurch gekennzeichnet, dass** das Chelatisierungsmittel ausgewählt ist aus Ethylendiamintetraessigsäure (EDTA) und/oder Ethylendiamindisuccinat (EDDS).

13. Kosmetisches Reinigungsmittel nach einem der vorherigen Ansprüche, **dadurch gekennzeichnet, dass** der Gewichtsanteil an Sulfatgruppen enthaltender Tenside am Gesamtgewicht der Zusammensetzung weniger als 0,5 Gew.-%, bevorzugt weniger als 0,3 Gew.-% besonders bevorzugt weniger als 0,2 Gew.-% beträgt.

## Claims

1. A cosmetic cleansing agent containing, in a suitable carrier,
a) at least one anionic surfactant according to the following formula (I), in which the functional groups R² to R⁵ each represent a hydrogen atom,
b) at least one anionic surfactant according to the following formula (I), in which at least one of the functional groups R² to R⁵ represents a C₁-C₄ alkyl functional group and the other functional groups independently of one another represent a hydrogen atom or a C₁-C₄ alkyl functional group, 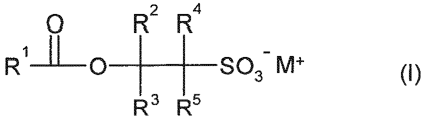 wherein
- R¹ in each case represents a linear or branched, saturated or unsaturated alkyl functional group having 6 to 30 carbon atoms, and
- M⁺ in each case represents an ammonium cation, an alkanol ammonium cation or a metal cation, and
c) at least one further surfactant, selected from
i. amphoteric surfactants of the following formula (II) 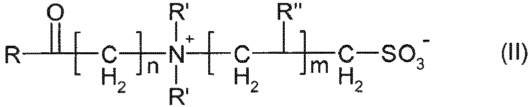 in which
- R represents a linear or branched, saturated or unsaturated alkyl functional group having 8 to 30 carbon atoms,
- R' represents a C₁-C₆ alkyl functional group or a C₁-C₆ hydroxyalkyl functional group,
- R" represents a hydrogen atom, a hydroxyl group, a C₁-C₆ alkyl functional group or a C₁-C₆ hydroxyalkyl functional group,
- the numbers n and m independently of one another represent numbers from 1 to 5, and/or
ii. alkyl(oligo)glycosides of general formula RO-[G]ₓ, in which R represents an alkyl functional group and/or an alkenyl functional group having 4 to 24 carbon atoms, G represents a sugar functional group having 5 or 6 carbon atoms and x represents numbers from 1 to 10.

2. The cosmetic cleansing agent according to claim 1, **characterized in that** the surfactants a) and b) are contained in a weight ratio of from 1:2.5 to 4:1, preferably from 1:2 to 3:1, particularly preferably from 1:1.5 to 2.5:1 and in particular preferably from 1:1 to 2:1.

3. The cosmetic cleansing agent according to one of the preceding claims, **characterized in that** it contains
a) at least one anionic surfactant according to formula (I), in which the functional groups R² to R⁵ each represent a hydrogen atom,
b) at least one anionic surfactant according to formula (I), in which at least one of the functional groups R² to R⁵ represents a methyl functional group and the other functional groups represent a hydrogen atom,
with the proviso that
- R¹ in each case represents a linear or branched, saturated or unsaturated alkyl functional group having 8 to 18 carbon atoms, and
- M⁺ in each case represents a sodium ion, potassium ion or ammonium ion.

4. The cosmetic cleansing agent according to one of the preceding claims, **characterized in that** it contains at least one of the anionic surfactants known by the INCI names sodium cocoyl isethionate or sodium lauroyl isethionate and at least one of the anionic surfactants known by the INCI names sodium lauroyl methyl isethionate or sodium cocoyl methyl isethionate.

5. The cosmetic cleansing agent according to one of the preceding claims, **characterized in that** the proportion by weight of the anionic surfactants a) and b) in the total weight of the composition is 0.5 to 20 wt.%, preferably 0.75 to 15 wt.%, particularly preferably 1 to 10 wt.% and in particular 1.5 to 7.5 wt.%.

6. The cosmetic cleansing agent according to one of the preceding claims, **characterized in that** it contains an amphoteric surfactant of formula (II) in a proportion by weight of from 1 to 15 wt.% in the total weight of the composition.

7. The cosmetic cleansing agent according to claim 1, **characterized in that**, in the surfactant according to formula (II),
- R represents a linear or branched, saturated or unsaturated alkyl functional group having 8 to 18 carbon atoms,
- R' represents a methyl or ethyl functional group, preferably a methyl functional group,
- R" represents a hydrogen atom or a hydroxyl group, preferably a hydroxyl group, and
- n represents a number from 1 to 3, preferably the number 3, and m represents the numbers 1 or 2, preferably the number 1.

8. The cosmetic cleansing agent according to one of the preceding claims, **characterized in that** it contains at least one anionic surfactant a) according to formula (I), at least one anionic surfactant b) according to formula (I) and at least one amphoteric surfactant according to formula (II), wherein
- the proportion by weight of surfactant a) in the total weight of the cleansing agents is preferably 0.5 to 20 wt.%, more preferably 0.75 to 15 wt.%,
- the proportion by weight of surfactant b) in the total weight of the cleansing agents is preferably 0.5 to 20 wt.%, more preferably 0.75 to 15 wt.%, and
- the proportion by weight of the surfactant according to formula (II) in the total weight of the cleansing agents is preferably 1 to 15 wt.%, more preferably 2 to 12.5 wt.%.

9. The cosmetic cleansing agent according to claim 1, **characterized in that** it contains an alkyl(oligo)glycoside of general formula RO-[G]ₓ in a proportion by weight of 0.5 to 10 wt.% in the total weight of the composition.

10. The cosmetic cleansing agent according to one of the preceding claims, **characterized in that** it contains at least one anionic surfactant a) according to formula (I), at least one anionic surfactant b) according to formula (I), at least one amphoteric surfactant according to formula (II) and at least one alkyl(oligo)glycoside,
- the proportion by weight of surfactant a) in the total weight of the cleansing agents preferably being 0.5 to 20 wt.%, more preferably 0.75 to 15 wt.%,
- the proportion by weight of surfactant b) in the total weight of the cleansing agents preferably being 0.5 to 20 wt.%, more preferably 0.75 to 15 wt.%,
- the proportion by weight of the surfactant according to formula (II) in the total weight of the cleansing agents preferably being 1 to 15 wt.%, more preferably 2 to 12.5 wt.%, and
- the proportion by weight of the alkyl(oligo)glycoside in the total weight of the cleansing agents preferably being 0.5 to 10 wt.%, more preferably 0.5 to 7.5 wt.%.

11. The cosmetic cleansing agent according to one of the preceding claims, **characterized in that** it additionally contains at least one chelating agent, the proportion by weight of the chelating agent in the total weight of the composition being 0.01 to 3 wt.%, preferably 0.05 to 2 wt.% and in particular 0.1 to 1 wt.%.

12. The cosmetic cleansing agent according to claim 11, **characterized in that** the chelating agent is selected from ethylenediaminetetraacetic acid (EDTA) and/or ethylenediamine disuccinate (EDDS).

13. The cosmetic cleansing agent according to one of the preceding claims, **characterized in that** the proportion by weight of surfactants containing sulfate groups in the total weight of the composition is less than 0.5 wt.%, preferably less than 0.3 wt.%, particularly preferably less than 0.2 wt.%.

## Revendications

1. Agent de nettoyage cosmétique contenant dans un support approprié
a) au moins un tensioactif anionique ayant la formule suivante (I), dans laquelle les radicaux R² à R⁵ représentent chacun un atome d'hydrogène,
b) au moins un tensioactif anionique ayant la formule suivante (I) dans laquelle au moins un des radicaux R² à R⁵ représentent un groupe alkyle en C₁ à C₄, et les autres radicaux représentent chacun indépendamment un atome d'hydrogène ou un groupe alkyle en C₁ à C₄, 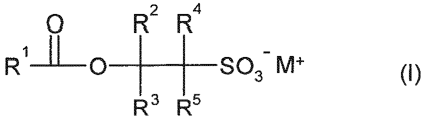 où
- R¹ représente à chaque fois un radical alkyle linéaire ou ramifié, saturé ou insaturé ayant 6 à 30 atomes de carbone, et
- M⁺ représente à chaque fois un ammonium, un alcanolammonium ou un cation métallique, et
c) au moins un autre tensioactif choisi parmi
i. les tensioactifs amphotères de la formule (II), 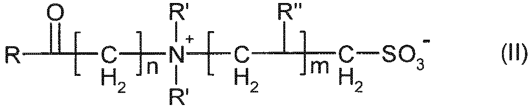 dans laquelle
- R représente un radical alkyle linéaire ou ramifié, saturé ou insaturé, ayant 8 à 30 atomes de carbone,
- R' représente un groupe alkyle en C₁ à C₆ ou un radical hydroxyalkyle en C₁ à C₆,
- R" représente un atome d'hydrogène, un groupe hydroxyle, un radical alkyle en C₁ à C₆ ou un radical hydroxyalkyle en C₁ à C₆,
- les nombres n et m représentent indépendamment des nombres de 1 à, 5 et/ou
ii. les alkyl(oligo)glycosides de la formule générale RO-[G]ₓ dans laquelle R représente un radical alkyle et/ou alcényle ayant 4 à 24 atomes de carbone, G représente un radical sucre ayant 5 ou 6 atomes de carbone et x représente un nombre de 1 à 10.

2. Agent de nettoyage cosmétique selon la revendication 1, **caractérisé en ce qu'**il contient les tensioactifs a) et b) dans un rapport en poids de 1:2,5 à 4:1, de préférence de 1:2 à 3:1, de manière particulièrement préférée de 1:1,5 à 2,5:1, et notamment de préférence de 1:1 à 2:1.

3. Agent de nettoyage cosmétique selon l'une des revendications précédentes, **caractérisé en ce qu'**il contient
a) au moins un tensioactif anionique ayant la formule (I) dans laquelle les radicaux R² à R⁵ représentent chacun un atome d'hydrogène,
b) au moins un tensioactif anionique ayant la formule (I) dans laquelle au moins un des radicaux R² à R⁵ représente un radical méthyle et les autres radicaux représentent un atome d'hydrogène,
à la condition que
- R¹ représente à chaque fois un radical alkyle linéaire ou ramifié, saturé ou insaturé, ayant 8 à 18 atomes de carbone, et
- M⁺ représente à chaque fois un ion sodium, potassium ou ammonium.

4. Agent de nettoyage cosmétique selon l'une des revendications précédentes, **caractérisé en ce qu'**il contient au moins un des tensioactifs anioniques connus sous les noms INCI Sodium Cocoyl isethionate ou Sodium Lauroyl Isethionate et au moins un des tensioactifs anioniques connus sous les noms INCI Sodium Lauroyl Methyl Isethionate ou Sodium Cocoyl Methyl Isethionate.

5. Agent de nettoyage cosmétique selon l'une des revendications précédentes, **caractérisé en ce que** la proportion en poids des tensioactifs anioniques a) et b) sur le poids total de la composition est de 0,5 à 20% en poids, de préférence de 0,75 à 15% en poids, de manière particulièrement préférée de 1 à 10% en poids et notamment 1,5 à 7,5% en poids.

6. Agent de nettoyage cosmétique selon l'une des revendications précédentes, **caractérisé en ce qu'**il contient un tensioactif amphotère de la formule (II) dans une proportion en poids de 1 à 15% en poids sur le poids total de la composition.

7. Agent de nettoyage cosmétique selon la revendication 1, **caractérisé en ce que** dans le tensioactif de la formule (II)
- R représente un radical alkyle linéaire ou ramifié, saturé ou insaturé, ayant 8 à 18 atomes de carbone,
- R' représente un radical méthyle ou éthyle, de préférence un radical méthyle,
- R" représente un atome d'hydrogène ou un groupe hydroxyle, de préférence un groupe hydroxyle, et
- n représente un nombre de 1 à 3, de préférence le nombre 3, et m représente le nombre 1 ou 2, de préférence le nombre 1.

8. Agent de nettoyage cosmétique selon l'une des revendications précédentes, **caractérisé en ce qu'**il contient au moins un tensioactif anionique a) ayant la formule (I), au moins un tensioactif anionique b) ayant la formule (I) et au moins un tensioactif amphotère ayant la formule (II),
- la proportion en poids du tensioactif a) sur le poids total de l'agent de nettoyage étant de préférence de 0,5 à 20% en poids, plus préférablement de 0,75 à 15% en poids,
- la proportion en poids du tensioactif b) sur le poids total de l'agent de nettoyage étant de préférence de 0,5 à 20% en poids, plus préférablement de 0,75 à 15% en poids, et
- la proportion en poids du tensioactif ayant la formule (II) sur le poids total de l'agent de nettoyage étant de préférence de 1 à 15% en poids, plus préférablement de 2 à 12,5% en poids.

9. Agent de nettoyage cosmétique selon la revendication 1, **caractérisé en ce qu'**il contient un alkyle(oligo)glycoside de la formule générale RO-[G]ₓ dans une proportion en poids de 0,5 à 10% sur le poids total de la composition.

10. Agent de nettoyage cosmétique selon l'une des revendications précédentes, **caractérisé en ce qu'**il contient au moins un tensioactif anionique a) ayant la formule (I), au moins un tensioactif anionique b) ayant la formule (I), au moins un tensioactif amphotère ayant la formule (II) et au moins un alkyl(oligo)glycoside,
- la proportion en poids du tensioactif a) sur le poids total de l'agent de nettoyage étant de préférence de 0,5 à 20% en poids, plus préférablement de 0,75 à 15% en poids,
- la proportion en poids du tensioactif b) sur le poids total de l'agent de nettoyage étant de préférence de 0,5 à 20% en poids, plus préférablement de 0,75 à 15% en poids,
- la proportion en poids du tensioactif ayant la formule (II) sur le poids total d'agent de nettoyage étant de préférence de 1 à 15% en poids, plus préférablement de 2 à 12,5% en poids, et
- la proportion en poids de l'alkyl(oligo)glycoside sur le poids total de l'agent de nettoyage étant de préférence de 0,5 à 10 .-%, plus préférablement de 0,5 à 7,5% en poids.

11. Agent de nettoyage cosmétique selon l'une des revendications précédentes, **caractérisé en ce qu'**il contient en outre au moins un agent chélatant, la proportion en poids de l'agent chélatant sur le poids total de la composition étant de 0,01 à 3% en poids, de préférence de 0,05 à 2% en poids et notamment de 0,1 à 1% en poids.

12. Agent de nettoyage cosmétique selon la revendication 11, **caractérisé en ce que** l'agent chélatant est choisi parmi l'acide éthylènediaminetétraacétique (EDTA) et/ou le disuccinate d'éthylènediamine (EDDS).

13. Agent de nettoyage cosmétique selon l'une des revendications précédentes, **caractérisé en ce que** la proportion en poids de tensioactifs contenant des groupes sulfate sur le poids total de la composition est inférieure à 0,5%, de préférence inférieure à 0,3%, de manière particulièrement préférée inférieure à 0,2%.
